# EUROPEAN PATENT SPECIFICATION

(11) **EP 2 351 846 B1**
(45) Date of publication and mention of the grant of the patent: **04.01.2017**
(21) Application number: 09823118.6
(22) Date of filing: 27.10.2009
(51) Int. Cl.: C12P 7/22, C12N 5/04

(54) **USE OF CYCLODEXTRINS FOR THE PRODUCTION AND EXTRACTION OF PHYTOSTEROLS IN CELL CULTURES**
VERWENDUNG VON CYCLODEXTRINEN ZUR HERSTELLUNG UND EXTRAKTION VON PHYTOSTEROLEN IN ZELLKULTUREN
UTILISATION DE CYCLODEXTRINES POUR LA PRODUCTION ET L'EXTRACTION DE PHYTOSTÉROLS DANS DES CULTURES CELLULAIRES

(30) Priority: 31.10.2008 ES 200803107
(43) Date of publication of application: 03.08.2011
(73) Proprietor: Universidad De Murcia, 30003 Murcia (ES); Universidad De Alicante, 03690 Alicante (ES)
(72) Inventor: SABATER JARA, Ana Belen, E-30003 Murcia (ES); ALMAGRO ROMERO, Lorena, E-30003 Murcia (ES); BRU MARTINEZ, Roque, Alicante (ES); PEDREÑO GARCIA, Mª Angeles, E-30003 Murcia (ES)
(74) Representative: Pons
(86) International application number: PCT/ES2009/070463
(87) International publication number: WO 2010/049563

(56) References cited:
- EP-A1- 1 471 141
- WO-A1-03/062406
- US-B1- 6 800 794
- LEE MI-HYUN ET AL: "Enhanced triterpene and phytosterol biosynthesis in Panax ginseng overexpressing squalene synthase gene", PLANT AND CELL PHYSIOLOGY, OXFORD UNIVERSITY PRESS, UK, vol. 45, no. 8, 1 August 2004 (2004-08-01) , pages 976-984, XP002424945, ISSN: 0032-0781, DOI: 10.1093/PCP/PCH126
- MANGAS S ET AL: "The effect of methyl jasmonate on triterpene and sterol metabolisms of Centella asiatica, Ruscus aculeatus and Galphimia glauca cultured plants", PHYTOCHEMISTRY, PERGAMON PRESS, GB, vol. 67, no. 18, 1 September 2006 (2006-09-01), pages 2041-2049, XP028059804, ISSN: 0031-9422, DOI: 10.1016/J.PHYTOCHEM.2006.06.025 [retrieved on 2006-09-01]
- SABATER-JARA ANA BELÉN ET AL: "Induction of sesquiterpenes, phytoesterols and extracellular pathogenesis-related proteins in elicited cell cultures of Capsicum annuum.", JOURNAL OF PLANT PHYSIOLOGY 15 OCT 2010 LNKD- PUBMED:20594613, vol. 167, no. 15, 15 October 2010 (2010-10-15), pages 1273-1281, XP002682674, ISSN: 1618-1328
- BRICEÑO ZULEIKA ET AL: "Enhancement of phytosterols, taraxasterol and induction of extracellular pathogenesis-related proteins in cell cultures of Solanum lycopersicum cv Micro-Tom elicited with cyclodextrins and methyl jasmonate.", JOURNAL OF PLANT PHYSIOLOGY 15 JUL 2012 LNKD- PUBMED:22608078, vol. 169, no. 11, 15 July 2012 (2012-07-15), pages 1050-1058, XP002682675, ISSN: 1618-1328
- MANGAS S. ET AL: 'The Effect of Methyl- Jasmonate on Triterpene and Sterol Metabolisms of Centel the asiatica, Ruscus aculeatus and Galphimia glauca Cultured Plants' PHYTOCHEMISTRY vol. 67, 2006, pages 2041 - 2049, XP025230984
- LEE M.H. ET AL: 'Enhanced Triterpene and Phytosterol Biosynthesis in Panax ginseng Overexpressing Squalene Synthase Gene' PLANT CELL PHYSIOL. vol. 45, no. 8, 2004, pages 976 - 984, XP002424945
- "Tropical Plant Breeding", 2001, Science Publishers Inc. ISBN: 1-57808-144-0 pages 25-26,
- Randhawa G.S. Mukhopadhyay A.: "Floriculture in India", 2004, Allied Publishers PVT. Limited ISBN: 81-7023-494-8 pages 29-30,
- Acram Taji, Prakash Kumar, Prakash Lakshmanan: "In Vitro Plant Breeding", 2002, Taylor & Francis ISBN: 156022908X pages 1-4,

## Description

The present invention is within the field of biotechnology and pharmacy, and it relates to a process to increase the production and/or extraction of phytosterols from cultures of plant cells by the addition to the culture medium of cyclodextrins and, optionally, methyl jasmonate.

### PRIOR ART

Phytosterols and phytostanols (reduced forms of phytosterols) are compounds derived from squalene whose common characteristic is the presence of a sterol ring, being differentiated in their lateral chains. Their main function is that of acting as structural components, since they are constituents of the plant cell membranes, although some phytosterols also have a hormonal action (Azcón-Bieto & Talón., 2000. Ed. McGraw-Hill).

Phytosterols belong to the family of triterpenes, they are sterols of plant origin whose chemical structure is very similar to that of cholesterol, which are present in the fruit, seeds, leaves and stems of practically all known fruit (Ling & Jones, 2005. Life Sci. 57:195-206). For this reason, we normally incorporate them in our diet. It is estimated that the daily average intake of phytosterols is in a range of 160 mg/day to 500 mg/day. The chemically identified phytosterols add up to more than 25 different structures, but only three are in greater proportion: β-sitosterol (C29), campesterol (C28) and stigmasterol (C29), which together constitute 95%-98% of the phytosterols identifiable in plant extracts. Phytosterols share with cholesterol the central nucleus of the molecule, the cyclopentane-perhydro-phenanthrene structure (D-5 unsaturated, conserving the -OH group which substitutes the carbon 3 of the cyclic structure). The structural difference of phytosterols with cholesterol lies in the lateral hydrocarbonated chain. In cholesterol this chain is formed by eight carbons and is saturated. In phytosterols, the lateral chain is longer (9 or 10 carbons) and in some of them has a double bond (stigmasterol). Phytostanols are in smaller proportion than phytosterols in the animal kingdom, they are formed by the reduction of the double bond of position D-5 of the cyclic structure. It has been proposed that the structural difference in the lateral chain of the phytosterols and of the phytostanols with the cholesterol is responsible for the particular hypocholesteremic effects.

Numerous experimental evidence have demonstrated that plant sterols have an important hypocholesteremic effect, reducing both total cholesterol levels and those of cholesterol linked to low density proteins (LDL) (Varady et al., 2007. Transl Res 149: 22-30.). The most and best studied effect of plant sterols is the inhibition of the intestinal absorption of cholesterol, both from the diet (300 mg/day) and the endogenous cholesterol circulating in the bile (100 mg/day) which, as it is partially but extensively reabsorbed in the intestine, constitutes the main form of uptake. Plant sterols, as they are more hydrophobic than cholesterol, may compete with it and displace it from the absorption micellae, having demonstrated, both in *in vivo* and *in vitro* studies that a decrease occurs in the incorporation of cholesterol in the micellae which translates into a decrease in the intestinal absorption of cholesterol. At high doses of plant sterols the absorption of cholesterol decreases 30-50% (Ostlund & Lin, 2006. Curr Atheroscler rep 8: 487-491). Furthermore, plant sterols may reduce the sterification rate of cholesterol in the enterocyte (affecting the activity of the enzymes involved) and consequently, in this way it will reduce the quantity of cholesterol exported in the blood in the form of chylomicrons. It has also been suggested that, in the presence of plant sterols, the cholesterols that is already in itself rather insoluble, can increase the rate of precipitation and, therefore, displace in non-absorbable or less absorbable form.

It is currently known that phytosterols and phytostanols have immunomodulator properties which can be beneficial for the prevention of colon cancer (McCann et al., 2003. J Nutr. 133:1937-1942), breast cancer (Awad et al., 2000. Anticancer Res. 20(2A):821-824.), control of benign prostate hyperplasia (Awad et al., 2001. Eur J Cancer Prev 10(6):507-513) and tissue damage associated to inflammation (Lowe & Ku, 1996. Urology 1996; 48:12-20).

Recent studies suggest that a diet rich in phytosterols may be highly protective against these types of cancer, triggering responses such as increase in antitumor response, inhibition of tumour growth; they also affect the cell cycle and promote apoptosis through induction of the sphingolipid metabolism and the stimulation of ceramide formation (Awad et al., 2000. Anticancer Res. 20(2A):821-824). Furthermore, the sterols increase the cellular response of T lymphocytes both *in vivo* and *in vitro* (Bouic et al., 1996. Int J Immunopharmacol. 18:693-700) and promote the expression of activity of endogenous factors inhibiting angiogenesis, a necessary process for the growth of tumours and development of metastasis (Quesada et al., 2006. Med. Res. Rev. 26:483-530). Likewise, phytosterols also have antimicrobial, antifungal and antibacterial action (Ling & Jones, 1995. Life Sci. 57:195-206).

The most important phytosterols, from a financial standpoint, are β-sitosterol, campesterol and stigmasterol. At present, these compounds are obtained by an extractive process of the plant raw material. This extraction process is low yield since 26.8 mg of a mixture of stigmasterol and β-sitosterol in a 2:1 proportion is obtained from 1.6 kg of dry *Pandanus tectorius* leaf (Mario et al., 2008. J Nat Med. 62:232-235). Likewise, from 325, 76, and 65 mg/kg of β-sitosterol, campesterol and stigmasterol respectively are obtained from 5 kg of banana fruit (Oliveira et al., 2008. J Agric Food Chem. 56(20):9520-9524).

*In vitro* culture constitutes an alternative to the classic methods of conventional extraction from raw plant material and it is an emerging area of great growth potential for the synthesis of bioactive compounds. The culture of plant cells *in vitro* has opened new pathways as a source for obtaining great added value due to the advantages its use has since these cultures are independent of geographic, seasonal and environmental factors, recognized as stable production systems, since they guarantee the continuous obtainment of compounds with uniform quality and productivity and the space requirements for the development of production are reduced. Furthermore, the purification process of the compound of interest is easy and optimized when it is released to the culture medium, the compound can be produced on a large scale and there is the possibility of obtaining new products that are not synthesized by plants naturally. The optimization of metabolite production includes establishing the optimum culture conditions and the adjustment of cell growth parameters. When, additionally, one wants to market the product industrially, it is necessary to perform a scaling in systems that guarantee the high production of the metabolite of interest (in biorreactors) to reach financially profitable levels. Furthermore, there are other factors that increase productivity among which we can highlight cell culture elicitation. Whilst the plant responds to the presence of elicitors by synthesizing metabolites in localized manner where the interaction occurs, the cell culture responds globally, since the *elicitor* comes into contact with all the biomass. Therefore, the total quantity of metabolites synthesized is much greater than in the plant and depends on the biomass of *elicited* cells (Bru et al., 2006. J Agr Food Chem. 54(1):65-71).

Lee M.-H. et al., Plant Cell Physiol.,45 (8): 976-984, refers to a process for producing phytosterols in ginseng plantlets by transgenic expression of a squalene synthase gene in roots. EP 1471141 A1 discloses that addition of cyclodextrin to plant cells in a culture medium results in production of resveratrol.

Due to the aforementioned beneficial properties, since 1997 margarines enriched with phytosterols have been sold in Finland, and currently in several countries in Europe there are dairy products enriched with them. Furthermore, since September 2000, the FDA has authorized the declaration on the packages of food rich in phytosterols of the benefit for health its consumption present in relation to a smaller risk of heart diseases. However, the cost of these products is high, and it is estimated that due to the great quantity of plant material necessary to extract a significant amount of phytosterols with the current natural sources, it would only be possible to supply 10% of the Western population (Law 2000. BMJ 320:861-864).

There is therefore the need to find a method that improves the yield of phytosterol extraction from natural resources, allowing a greater percentage of the population to benefit from the positive effects said phytosterols have on the health.

### DESCRIPTION OF THE INVENTION

The present invention provides a process for the production and extraction of phytosterols with high yields, wherein the process is characterised by the steps (a) to (d) as mentioned bellow.

The authors of the present invention have observed that establishing cultures of plant cells and adding cyclodextrins and, optionally, methyl jasmonate, to the medium increases the phytosterol production and extraction yield.

Therefore, a first aspect of the invention relates to a process to increase the production and/or extraction of phytosterols comprising:
a) adding cyclodextrins to a culture medium,
b) contacting plant cells with the potential to produce phytosterol with the culture medium of a),
c) incubating the cells of step b) in the culture medium of step a),
d) separating the phytosterols obtained after step c) of the culture medium.

Suitable culture media are known in the state of the art. Thus, and without limiting the scope of the invention, a suitable culture medium is described for each species used in the examples of the invention. To this medium can be added other components depending on the species the plant cells belong to. The cyclodextrins are incorporated in the culture medium at the time of their preparation.

The plant cells with the potential to produce phytosterol may come from leaves, tissues and organs that have been established, previously or not, in *in vitro* cultures. Therefore, plant cells with the potential to produce phytosterol are understood to be any cell line capable of producing phytosterols, either naturally or after genetic modification.

Any plant organ, tissue or cell capable of producing phytosterols may be used in the invention. This includes those cells from an organism which, although it does not naturally have the capacity to synthesize phytosterols, has acquired said capacity by genetic manipulation processes. In a preferred embodiment, they are plant tissues or cells from plants of the genera selected from the list comprising: *Rosa, Daucus, Capsicum, Lactuca, Catharanthus, Lycopersicon, Taxus* and *Vitis.*

The term "plant" or "organism" includes parts, tissues, cells or protoplasts from the plant or organism, cell cultures, tissue cultures, calluses, ovules, embryos and seeds in last instance from the plant or organism.

Taxonomically the organisms of the *Rosa* genus are defined as cellular organisms, which belongs to the superkingdom *Eukaryota,* kingdom *Viridiplante,* phylum *Streptophyta,* order *Rosales,* family *Rosaceae,* Subfamily *Rosoideae.*

Taxonomically the organisms of the *Daucus* genus are defined as cellular organisms, which belongs to the superkingdom *Eukaryota,* kingdom *Viridiplante,* phylum *Streptophyta,* order *Apiales,* family *Apiaceae,* Subfamily *Apioideae.*

Taxonomically the organisms of the *Daucus* genus are defined as cellular organisms, which belongs to the superkingdom *Eukaryota,* kingdom *Viridiplante,* phylum *Streptophyta,* order *Apiales,* family *Apiaceae,* Subfamily *Apioidea.*

Taxonomically the organisms of the *Capsicum* genus are defined as cellular organisms, which belongs to the superkingdom *Eukaryota,* kingdom *Viridiplante,* phylum *Streptophyta,* order *Solanales,* family *Solanaceae,* Subfamily *Solanoideae.*

Taxonomically the organisms of the *Lactuca* genus are defined as cellular organisms, which belongs to the superkingdom *Eukaryota,* kingdom *Viridiplante,* phylum *Streptophyta,* order *Asterales,* family *Asteraceae,* Subfamily *Cichorioideae.*

Taxonomically the organisms of the *Catharanthus* genus are defined as cellular organisms, which belongs to the superkingdom *Eukaryota,* kingdom *Viridiplante,* phylum *Streptophyta,* order *Gentianales,* family *Apocynaceae,* Subfamily *Rauvolfioideae.*

Taxonomically the organisms of the *Lycopersicum* genus are defined as cellular organisms, which belongs to the superkingdom *Eukaryota,* kingdom *Viridiplante,* phylum *Streptophyta,* order *Solanales,* family *Solanaceae,* Subfamily *Solanoideae.*

Taxonomically the organisms of the *Taxus* genus are defined as cellular organisms, which belongs to the superkingdom *Eukaryota,* kingdom *Viridiplante,* phylum *Streptophyta,* order *Coniferales,* family *Taxaceae.*

Taxonomically the organisms of the *Vitis* genus are defined as cellular organisms, which belongs to the superkingdom *Eukaryota,* kingdom *Viridiplante,* phylum *Streptophyta,* order *Vitales,* family *Vitaceae.*

In this way, cultures of potential phytosterol-producing cells are produced in suitable culture media which have been enriched with cyclodextrins. The term "cell culture" in this specification makes references to an isolated cell culture of the same or different type of tissue, preferably of a plant, a collection of said cells organized in parts of a plant in tissues (tissue cultures). Types of cultures of this type are, for example, cultures of protoplasts, calluses (group of undifferentiated plant cells capable of regenerating a complete plant) and plant cells that are isolated from plants or parts of the plants, such as embryos, protoplasts, which are isolated from plants or parts of the plants, such as embryos, protoplasts, meristematic cells, pollen, leaves, roots, inclined root, anther, pistil, flower, seed, pod or shoot of the plant.

Potential phytosterol-producing cells are also understood to be those that come from vitroplants, organs or tissues of said vitroplants, and specifically, from vitroplants, organs or tissues of vitroplants of the genera selected from the list comprising: *Rosa, Daucus, Capsicum, Lactuca, Catharanthus, Lycopersicon, Taxus* and *Vitis.*

Thus, in another preferred embodiment of this aspect of the invention, the potential phytosterol-producing cells come from vitroplants.

The cyclodextrins are cyclic oligosaccharides that are formed in some starch degradation processes. Sometimes they are also called cycloamyloses. They have a hydrophilic exterior and a hydrophobic interior cavity where non-polar molecules can be trapped. To change the physical and chemical properties of the cyclodextrins several derivatives have been developed. One of the most widely used are the partially methylated derivatives which have solubility in water up to 150 times greater than the starting product.

Thus, in another preferred embodiment, the cyclodextrins added in step a) of the process of the invention are chosen from the group comprising randomly methylated cyclodextrin (CDMA) or hydroxypropylated cyclodextrin (CDHA). Preferably, the degree of substitution of the methyls per glucose unit (anhydrous) of the CDMA is between 1 and 3, and more preferably, the degree of substitution by methyls per glucose unit (anhydrous) of the CDMA is 2.

The randomly methylated cyclodextrin can be dimethylated.

In another preferred embodiment of the invention, the randomly hydroxypropylated cyclodextrin has a degree of substitution by hydroxypropyls of between 0.6 and 0.9.

In another preferred embodiment of this aspect of the invention the cyclodextrin is a cyclic maltooligosaccharide consisting of D-glucose bound by type α glycosidic bonds (1 → 4) (β-cyclodextrins).

In another preferred embodiment the concentration of cyclodextrins in the culture medium of step (a) of the process of the invention is between 6.5 and 130 g/L of culture medium. More preferably the concentration of cyclodextrins is between 10 and 100 g/L culture medium and even more preferably is between 50 and 75 g/L culture medium.

Methyl jasmonate is a growth regulator, modulating multiple aspects of plant development (ripening of fruit, pollen viability, root growth, tendril curvature) and which promotes foliage aging, producing senescence. It seems to act specifically on the expression of genes involved in the defence of plants to insect attacks and pathogens and genes that code for reserve proteins.

In another preferred embodiment, in addition to adding cyclodextrins, methyl jasmonate is added to the culture medium of step a) of the process of the invention. In another more preferred embodiment of the present invention the concentration of methyl jasmonate in the culture medium of step (a) is between 5 and 500 micromoles per litre of culture medium. More preferably the concentration of methyl jasmonate is between 25 and 150 micromoles/L of culture medium and even more preferably of between 75 and 125 micromoles/L of culture medium.

Another aspect of the invention relates to the use of a composition, hereinafter composition of the invention, comprising cyclodextrins to increase the production and/or extraction of phytosterols in plant cells with the potential to produce phytosterol. In a preferred embodiment, the composition of the invention comprises, in addition to cyclodextrins, methyl jasmonate. Another aspect of the invention relates to the use of a culture medium, hereinafter culture medium of the invention, comprising cyclodextrins to increase the production and/or extraction of phytosterols in plant cells with the potential to produce phytosterol. In a preferred embodiment, the culture medium of the invention comprises in addition to cyclodextrins, methyl jasmonate.

Both the cell density in the culture medium and the photoperiod may vary depending on the origin of the potential phytosterol-producing cells. The photoperiod will preferably be adjusted to a natural cycle of between 12-16 hours of light and 8-12 hours of dark.

In the description and in the claims, the word "comprise" and its variants do not intend to exclude other technical characteristics, additives, components or steps. For persons skilled in the art, other objects, advantages and characteristics of the invention can be inferred in part from the description and in part from the practice of the invention.

### DETAILED EXPLANATION OF EMBODIMENTS

Below, the invention will be illustrated by assays performed by the inventors, which reveal the effect of the use of cyclodextrins and methyl jasmonate in the production of phytosterols from cell suspensions of different plant species.

### Preparation and maintenance of plant material

### ➢Cell line: Rosa sp.

The Rosa mini (*Rosa sp.*) cell line was started from leaves of plants grown in the greenhouses of Viveros Bermejo S.L. For this purpose, the leaves were washed three times with soapy water. Later, the disinfection was carried out with 70% ethanol during 1 minute and then they were immersed for 15-20 minutes in a 10% sodium hypochlorite solution containing 0.1% Tween 20. After this time, the leaves were washed three times with sterile distilled water working from this wash and in the following stages in the laminar flow cabin.

Once disinfected they were cut into fragments of 1.5-2.0 mm and they were deposited on petri dishes containing an optimum culture medium for induction of calluses based on that described by Murashige & Skoog, 1962 (Physiol. Plant. 15:473-497), supplemented with calcium pantothenate (1mg/l), myo-inositol (100mg/l), biotin (0.01 mg/l), pyridoxine (1 mg/l), thiamine (1 mg/l), nicotinic acid (1 mg/l). As a carbonated source, sucrose (30 g/l) was used and 2.4-dichlorophenoxyacetic (1.1 mg/l) and benzyladenine (0.45 mg/l) as hormones. This medium is adjusted to pH 6 and it is sterilized by the application of humid heat (autoclave) during 20 min at 1.2 atm of pressure acquiring solid consistency by the addition of purified agar (8 g/l), when the medium is cooled.

The cell suspensions were started, from the friable calluses obtained, in 250 ml-capacity volumetric flasks containing 80 ml of the culture medium described without agar. The cells in liquid medium were maintained in an orbital stirrer (100 rpm), at 25ºC under a 16 h photoperiod of light, 8 of dark and were subcultivated every 14-16 days.

### ➢ Cell line: Daucus carota

### Cell line: Daucus carota L. var. sativus (Hoffm.) Arcang PC-1106

### Cell line: Daucus carota L. cv. Chantenay.

The cell line *Daucus carota* L. var. *sativus* (Hoffm.) Arcang PC-1106 was obtained from German Collection of Microorganisms and Cell Cultures (DSMZ; Braunschweig, Germany).

For the induction of calluses of *Daucus carota* L. cv. Chantenay, vitroplants were used which were obtained by *in vitro* germination of seeds.

### ▪ in vitro germination of Daucus carota seeds, Chantenay variety.

The main objective of the *in vitro* germination of seeds is to obtain sterile plants for their use in the induction of cell lines of the different plant organs: root, stalk and leaf.

To obtain the carrot vitroplant seeds were used supplied by Viveros Bermejo S.L. The seeds were subjected to a disinfection process with 70% ethanol for 1 minute and they were then immersed for 15-20 minutes in a 7% calcium hypochlorite solution containing 0.1% Tween 20. After this time, the seeds were washed three times with sterile distilled water working from this wash and in the following stages in the laminar flow cabin.

The disinfected seeds were transferred to tubes containing the culture medium based on that described by Murashige & Skoog, 1962 (Physiol. Plant. 15:473-497), supplemented with calcium pantothenate (1mg/l), myo-inositol (100mg/l), biotin (0.01 mg/l), pyridoxine (1 mg/l), thiamine (1 mg/l), nicotinic acid (1 mg/l) and casein hydrolysate (300 mg/L). As a carbonated source, sucrose was used (25 g/l). This medium is adjusted to pH 5.8 and it is sterilized by the application of humid heat (autoclave) during 20 min at 1.2 atm of pressure, acquiring solid consistency by the addition of purified agar (8 g/l), when the medium is cooled.

### ▪ Obtainment of calluses and maintenance of Daucus carota cell suspensions Chantenay variety.

The stalks of the vitroplants were used as a source of explants. The petri dishes with the explants were kept under a photoperiod of 16 hours of light, 8 hours of dark at an irradiance of 4.6 w/m² at 25 ºC, observing after 2-3 weeks the appearance of microcalluses which were transferred to 250 ml-capacity volumetric flasks containing 100 ml of the aforementioned culture medium supplemented with naphthaleneacetic acid (2 mg/l) and benzyladenine (0.2 mg/l).

From the cell line generated under the described conditions a cell line was originated in the dark in order to compare the production of phytosterols in light and dark.

The cell suspensions were started, from friable calluses, in 250 ml- capacity volumetric flasks containing 80 ml of culture medium without agar. The cells in liquid medium were maintained in an orbital stirrer (100 rpm), in the same conditions of light and Temperature and were subcultivated every 20 days.

### ➢ Cell line: Capsicum annuum and Capsicum chinense

For the induction of calluses of *Capsicum,* vitroplants obtained from seeds germinated *in vitro* were used.

### ▪ in vitro germination of Capsicum seeds

To obtain the *Capsicum* vitroplant seeds were used that were subjected to a disinfection process with 70% ethanol for 2 minutes and they were then immersed for 20 minutes in a 20% sodium hypochlorite solution containing 0.1% Tween 20. After this time, the seeds were washed three times with sterile distilled water, working from this wash and in the following stages in the laminar flow cabin.

The disinfected seeds were transferred to tubes containing the culture medium based on that described by Murashige & Skoog, 1962 (Physiol. Plant. 15:473-497), supplemented with calcium pantothenate (1 mg/l), myo-inositol (100mg/l), biotin (0.01 mg/l), pyridoxine (1 mg/l), thiamine (1 mg/l), nicotinic acid (1 mg/l) and casein hydrolysate (300 mg/L). As a carbonated source, sucrose was used (25 g/l). This medium is adjusted to pH 5.8 and it is sterilized by the application of humid heat (autoclave) during 20 min at 1.2 atm of pressure, acquiring solid consistency by the addition of purified agar (8 g/l), when the medium is cooled. The *in vitro* culture tubes were maintained in a germination chamber for 15 days, at 25º C under a photoperiod of 16 hours of light and 8 hours of dark.

### ▪ Obtainment of calluses and maintenance of Capsicum cell suspensions.

Once the seedlings had grown hypocotyls were used as source of explant, for the obtainment of the microcalluses in the aforementioned culture medium supplemented with naphthaleneacetic acid (2 mg/l) and benzyladenine (0.2 mg/l). The explants were kept under a photoperiod of 16 hours of light, 8 hours of dark at an irradiance of 4.6 w/m² at 25 ºC, observing after 2 weeks the appearance of these microcalluses which were transferred to 250 ml-capacity volumetric flasks containing 100 ml of the same culture medium.

The cell suspensions were started, from friable calluses, in 250 ml- capacity volumetric flasks containing 80 ml of culture medium without agar. The cells in liquid medium were maintained in an orbital stirrer (100 rpm) in the same conditions of light and temperature and were subcultivated every 20 days.

### ➢ Cell line: Lactuca sativa

For the induction of *Lactuca sativa* calluses leaves of vitroplants obtained by *in vitro* germination were used.

### ▪ in vitro germination of Lactuca sativa seeds

To obtain the *Lactuca sativa* vitroplants seeds were used supplied by Ramiro Arnedo S.L. The seeds were subjected to a disinfection process with 70% ethanol for 1 minute and they were then immersed for 15 minutes in a 7% calcium hypochlorite solution containing 0.1% Tween 20. After this time, the seeds were washed three times with sterile distilled water working from this wash and in the following stages in the laminar flow cabin.

The disinfected seeds were transferred to tubes containing the culture medium based on that described by Murashige & Skoog, 1962 (Physiol. Plant. 15:473-497), supplemented with calcium pantothenate (1 mg/l), myo-inositol (100mg/l), biotin (0.01 mg/l), pyridoxine (1 mg/l), thiamine (1 mg/l), nicotinic acid (1 mg/l) and casein hydrolysate (300 mg/L). As a carbonated source, sucrose was used (25 g/l). This medium is adjusted to pH 6 and it is sterilized by the application of humid heat (autoclave) during 20 min at 1.2 atm of pressure, acquiring solid consistency by the addition of purified agar (8 g/l), when the medium is cooled.

### ▪ Obtainment of calluses and maintenance of Lactuca sativa cell suspensions

Once the seedlings had grown the leaves and the root were used as source of explants for the obtainment of the microcalluses in petri dish. The petri dishes with the explants were kept under a photoperiod of 16 hours of light, 8 hours of dark at an irradiance of 4.6 w/m² at 25 ºC, observing after 2-3 weeks the appearance of microcalluses which were transferred to 250 ml- capacity volumetric flasks containing 100 ml of the culture medium described above supplemented with naphthaleneacetic acid (1 mg/l) and kinetin (1 mg/l).

The cell suspensions were started, from portions of friable calluses, in 250 ml-capacity volumetric flasks containing 80 ml of culture medium without agar. The cells in liquid medium were maintained in an orbital stirrer (100 rpm), at 25ºC in the conditions described above and were subcultivated every 16 days.

### ➢ Cell line: Catharanthus roseus

The *C. roseus* (L.) G.Don cell line was obtained from German Collection of Microorganisms and Cell Cultures (DSMZ; Braunschweig, Germany, number or PC-1101).

The calluses were kept at 25ºC in the dark, in 250 ml-capacity volumetric flasks containing 100 ml of a medium containing LS basal medium (Linsmaier & Skoog, F. 1965 Physiol. Plant. 18, 100-127) supplemented with thiamine (0.4 mg/l) and myo-inositol (100 mg/l) and sucrose (30 g/l) As a carbonated source, alpha-naphthaleneacetic acid (0.19 mg/l) and 2.4-dichlorophenoxyacetic (0.22 mg/l) as hormones. This medium is adjusted to pH 6 and it is sterilized by the application of humid heat (autoclave) during 20 min at 1.2 atm of pressure, acquiring solid consistency by the addition of purified agar (8 g/l), when the medium is cooled.

The cell suspensions were started, from friable calluses, in 250 ml- capacity volumetric flasks containing 80 ml of the LS medium without agar. The cells in liquid medium were maintained in an orbital stirrer (100 rpm), at 25ºC in the dark.

### ➢ Cell line: Lycopersicon esculentum

### Cell line: Lycopersicon esculentum, Micro-Tom variety

### Cell line: Lycopersicon esculentum, Durinta variety

The cell lines derived from the Durinta tomato supplied by the Wester Seed (Netherlands) were obtained from different tissues (epidermis and pericarp) of the fruit.

For this, the fruit was washed three times with soapy water. It was then disinfected with 70% ethanol for 1 minute and 7% calcium hypochlorite for 20 minutes. After the disinfection, it was rinsed with abundant sterile distilled water. Once disinfected, explants of 1.5-2.0 mm were selected and they were deposited on petri dishes containing an optimum culture medium for the induction of calluses based on that described by Murashige and Skoog (1962), supplemented with calcium pantothenate (1 mg/l), myo-inositol (1 00mg/l), biotin (0.01 mg/l), pyridoxine (1 mg/l), thiamine (1 mg/l), nicotinic acid (1 mg/l) and casein hydrolysate (250 mg/l); As a carbonated source, sucrose was used (30 g/l) and 2.4-dichlorophenoxyacetic (1 mg/l) as hormone. This medium is adjusted to pH 6 and it is sterilized by the application of humid heat (autoclave) during 20 min at 1.2 atm of pressure, acquiring solid consistency by the addition of purified agar (7.5 g/l) when the medium is cooled.

The cell suspensions were started, from friable calluses, in 250 ml- capacity volumetric flasks containing 80 ml of culture medium without agar. The cells in liquid medium were maintained in an orbital stirrer (100 rpm), at 25ºC under a 16 h photoperiod of light, 8 of dark and were subcultivated every 14-16 days.

### Cell line: Lycopersicon esculentum, Micro-Tom variety

For the induction of calluses of *Lycopersicon esculentum,* Micro-Tom variety, vitroplants were used obtained by *in vitro* germination.

### ▪ in vitro germination of Lycopersicon esculentum seeds, Micro-Tom variety

To obtain the *Lycopersicon esculentum* vitroplants seeds were used supplied by Ramiro Arnedo S.L. The seeds were subjected to a disinfection process with 70% ethanol for 1 minute and they were then immersed for 15 minutes in a 7% calcium hypochlorite solution containing 0.1 % Tween 20. After this time, the seeds were washed three times with sterile distilled water working from this wash and in the following stages in the laminar flow cabin.

The disinfected seeds were transferred to tubes containing the culture medium based on that described by Murashige & Skoog, 1962 (Physiol. Plant. 15:473-497), supplemented with calcium pantothenate (1 mg/l), myo-inositol (100mg/l), biotin (0.01 mg/l), pyridoxine (1 mg/l), thiamine (1 mg/l), nicotinic acid (1 mg/l) and casein hydrolysate (250 mg/l). As a carbonated source, sucrose was used (30 g/l). This medium is adjusted to pH 6 and it is sterilized by the application of humid heat (autoclave) during 20 min at 1.2 atm of pressure, acquiring solid consistency by the addition of purified agar (8 g/l), when the medium is cooled.

### ▪ Obtainment of calluses and maintenance of Lycopersicon esculentum cell suspensions, Micro-Tom variety

Once the seedlings had grown the axillary leafbuds were used as source of explant, for the obtainment of the microcalluses in the aforementioned culture medium supplemented with naphthaleneacetic acid (2 mg/l) and benzyladenine (2 mg/l). The explants were kept under a photoperiod of 16 hours of light, 8 hours of dark at an irradiance of 4.6 w/m² at 25 ºC, observing after 2 weeks the appearance of these microcalluses which were transferred to 250 ml-capacity volumetric flasks containing 100 ml of the same culture medium.

The cell suspensions were started, from portions of friable calluses, in 250 ml-capacity volumetric flasks containing 80 ml of the culture medium without agar. The cells in liquid medium were maintained in an orbital stirrer (100 rpm), at 25ºC in the conditions described above and were subcultivated every 16 days.

### ➢ Cell line: Taxus sp.

The *Taxus sp* cell line was started from leaves of plants cultivated in the greenhouses of Viveros Bermejo S.L. For this, the leaves were washed three times with soapy water. It was then disinfected with 70% ethanol for 1 minute and they were then immersed for 20 minutes in a 8% calcium hypochlorite solution containing 0.1% Tween 20. After this time, the leaves were washed three times with sterile distilled water working from this wash and in the following stages in the laminar flow cabin.

Once the leaves were disinfected they were cut in 1.5-2.0 mm fragments and they were deposited on petri dishes containing an optimum culture medium for the induction of calluses containing Gamborg salts (3.05 g/l) supplemented with calcium pantothenate (1 mg/l), myo-inositol (100 mg/l), biotin (0.01 mg/l), pyridoxine (1 mg/l), thiamine (1 mg/l), nicotinic acid (1 mg/l) and casein hydrolysate (500mg/l). As a carbonated source, sucrose was used (30 g/l) and 2.4-dichlorophenoxyacetic (2 mg/l) and kinetin (0.2 mg/l) as hormones. This medium is adjusted to pH 5.8 and it is sterilized by the application of humid heat (autoclave) during 20 min at 1.2 atm of pressure, acquiring solid consistency by the addition of purified agar (8 g/l), when the medium is cooled.

The cell suspensions were started from friable calluses, in 250 ml- capacity volumetric flasks containing 80 ml of the culture medium without agar. The cells in liquid medium were maintained in an orbital stirrer (100 rpm), at 25ºC under a 16 h photoperiod of light, 8 of dark and were subcultivated every 20 days.

### ➢ Cell line: Vitis vinifera cv. Monastrell

The *Vitis vinifera* cv. Monastrell cell line were obtained from immature fruit of this vine culture. For this, the immature fruit, of approximately 5 mm diameter were superficially sterilized by immersion in a 7% calcium hypochlorite solution for 15 minutes. After this time, and always under sterile conditions, the berries were washed three times with sterile distilled water, the seeds were eliminated and they were divided in four portions. Each of these portions were deposited in a petri dish containing a culture medium based on that described by Murashige & Skoog, 1962 (Physiol. Plant. 15:473-497), supplemented with vitamins, hormones, casein hydrolysate, sucrose and agar, adjusting the pH to 6.0.

The dishes with the explants were kept in the dark at 25ºC, observing after 2-3 weeks the appearance of microcalluses, which were transferred to 250 ml-capacity volumetric flasks containing 100 ml of the medium described above. The friable calluses obtained were subcultivated every three weeks.

Then, these calluses were transferred to Gamborg B5 culture medium, supplemented with kinetin (0.2 mg/l), naphthaleneacetic acid (0.1 mg/l), casein hydrolysate (250 mg/l) and sucrose (20 g/l). The calluses were kept friable in this state in conditions for the obtainment of cell suspensions, in the dark at 25ºC.

The cell suspensions were started by the transfer of portions of friable callus in 250 ml-capacity volumetric flasks, containing 100 ml of Gamborg liquid culture medium described above. After several subcultures of the suspensions in this volume, it was scaled in 500 ml capacity volumetric flasks containing 200 ml of culture medium. They were kept under stirring at 105 rpm, in identical dark and temperature conditions to those described for the above and they were subcultivated every 14-16 days.

### Cell elicitation

In each elicitation experience, in conditions of sterility, between 420 and 110 grams of fresh weight of cells per litre were taken, which had been previously washed with fresh medium and filtered. Using this cell density, the cells were distributed in 250 ml volumetric flasks containing 100 ml of fresh medium:
- only with a randomly methylated ß- cyclodextrin with a degree of substitution by methyls of between 1.6 and 1.9 (CDMA) at a concentration of 62.5 g/l.
- randomly hydroxypropylated with a degree of substitution by hydroxypropyls of between 0.6 and 0.9 (CDHA), at a concentration of 62.5 g/l.
- with one of the aforementioned cyclodextrins (CDMA) or (CDHA) at a concentration of 62.5 g/l and with methyl jasmonate at a concentration of 100 micromolar.

The methyl jasmonate is sterilized by filtration, dissolved in ethanol and it is later mixed with the rest of the sterile medium. The final concentration of ethanol in the culture medium is 0.043 moles per litre.

The volumetric flasks were incubated in the same conditions described above for its maintenance in liquid medium for 96 hours.

### Sampling and preparation of samples for analysis

The cell cultures incubated with elicitors were collected 96 hours after treatment for their analysis.

In the case of cell suspensions, the cells were separated from the medium by filtration performing a slight vacuum, separately collecting the cells and the filtrate. The filtrate was used for the extraction of the compounds and later analysis by gas/mass chromatography.

### Analysis of phytosterols in the extracellular medium

Once the elicitation experiments were performed, the extraction of the compounds secreted to the culture medium of the different cell line was carried out by phase partition between ethyl acetate and water containing 3% NaHCO₃ (w/v) and 10% NaCl (w/v). The organic phase was collected and evaporated in a rotary evaporator at 40 °C under vacuum; the dry residue was resuspended in 1 ml of methanol and it was analysed in the gas/mass chromatograph.

The assays were performed using a Agilent Technologies 6890 Network GS System chromatographic system equipped with a Agilent 5973 mass detector. The separation of the compounds was carried out on an Agilent 19091 S-433 HP-5MS capillary column, with a flow of 1.0 ml/min of helium as carrier gas. The injector temperature was adjusted to 250 °C and the injection volume was 1 µl. The analysis program is as follows: it starts at 60 °C rising to 310 °C reaching a maximum temperature of 350 °C. The identification of the different compounds was carried out by the combination of the analysis of the mass spectrometer and the retention times.

The retention time of the different phytosterols and the response of the detector at the concentration thereof (calibration curve for quantification) was determined using external standards.

### EXAMPLE 1: Elicitation of Lycopersicon esculentum cells, Durinta variety, obtained from epidermis of the fruit with cyclodextrins (CDMA and CDHA) and methyl jasmonate (MeJA) using a high cell density (260g pf/l, 9.2g ps/l).

The suspensions of *Lycopersicon esculentum* were treated with:
- without CDMA or MeJA (controls)
- with MeJA (100 micromoles/l)
- with CDMA (62.5 g/l)
- with CDMA (62.5 g/l) and MeJA (100 micromoles/l)
- with CDHA (62.5 g/l)
- with CDHA (62.5 g/l) and MeJA (100 micromoles/l)

The experiments were carried out in triplicate in 250 ml volumetric flasks containing 100 ml of culture medium. After 96 hours, the cells and medium were collected separately from each volumetric flask, the cells were weighed and the total quantity of phytosterols in the extracellular medium was determined. The result of the experiment is shown in Table A.

The cultures elicited only with CDs or in combination with MeJA, induce greatest extracellular accumulation of stigmasterol and treatment with CDHA+MeJA being that which produced a greatest increase in production with respect to the control. Likewise, the treatment that caused the greatest increase in fucosterol biosynthesis was CDHA with MeJA.

On the other hand, it was observed that there were no significant differences in the production of β-sitosterol in the cultures treated with MeJA, CDMA and the combination of both after 96 hours of elicitation. Only the treatments with CDHA and the combination of CDHA with MeJA were translated into an increase in β-sitosterol production.

These results indicate that the treatment that produced greatest production of phytosterols in this cell line is the combination of CDHA and MeJA.

**TABLE A: Phytosterol production expressed in mg/l of β-sitosterol of Lycopersicon esculentum after 96 hours of treatment. Cell density: 9.2g ps/l.**

| **Compound** | **Control** | **MeJA** | **CDMA** | **CDMA+MeJA** | **CDHA** | **CDHA+ MeJA** |
|---|---|---|---|---|---|---|
| **stigmasterol** | 0 | 3 | 6 | 5 | 14 | 28 |
| **β-sitosterol** | 3 | 3 | 4 | 3 | 6 | 11 |
| **fucosterol** | 4 | 5 | 26 | 11 | 38 | 67 |

Control: culture medium without elicitors
MeJA: treatment with 100 micromoles/l of MeJA
CDMA: treatment with 62.5g/l of CDMA
CDMA+MeJA: treatment with 62.5g/l of CDMA combined with 100 micromoles/l of MeJA
CDHA: treatment with 62.5g/l of CDHA
CDHA+MeJA: treatment with 62.5g/l of CDHA combined with 100 micromoles/l of MeJA

### EXAMPLE 2: Elicitation of Lycopersicon esculentum cells, Durinta variety, obtained from the pericarp of the fruit with cyclodextrins (CDMA and CDHA) and methyl jasmonate (MeJA) using a high cell density (260g pf/l, 6g ps/l).

The suspensions of *Lycopersicon esculentum* were treated with:
- controls without CDMA or MeJA
- with MeJA (100 micromoles/l)
- with CDMA (62.5 g/l)
- with CDMA (62.5 g/l) and MeJA (100 micromoles/l)
- with CDHA (62.5 g/l)
- with CDHA (62.5 g/l) and MeJA (100 micromoles/l)

The experiments were carried out in triplicate in 250 ml volumetric flasks containing 100 ml of culture medium using a high cell density (260 g/pf/l). After 96 hours, the cells and medium were collected separately from each volumetric flask, the cells were weighed and the total quantity of phytosterols in the extracellular medium was determined. The result of the experiment is shown in Table B.

Of all the assays performed, the use of CDHA alone or in combination with MeJA was the treatment that induced the greatest increase in the extracellular accumulation of stigmasterol, β-sitosterol and fucosterol.

In terms of taraxasterol production, no significant differences were observed with respect to the control.

**TABLE C: Phytosterol production expressed in mg/l of β-sitosterol of Lycopersicon esculentum after 96 hours of incubation. Cell density: 6g ps/l.**

| **Compounds** | **Control** | **MeJA** | **CDMA** | **CDMA+MeJA** | **CDHA** | **CDHA+MeJA** |
|---|---|---|---|---|---|---|
| **stigmasterol** | 3 | 3 | 7 | 9 | 16 | 19 |
| **β-sitosterol** | 0 | 4 | 6 | 7 | 12 | 10 |
| **fucosterol** | 3 | 4 | 15 | 20 | 39 | 31 |
| **taraxasterol** | 9 | 9 | 4 | 6 | 5 | 5 |

Control: culture medium without elicitors.
MeJA: treatment with 100 micromoles/l of MeJA.
CDMA: treatment with 62.5g/l of CDMA.
CDMA+MeJA: treatment with 62.5g/l of CDMA combined with 100 micromoles/l of MeJA.
CDHA: treatment with 62.5g/l of CDHA.
CDHA+MeJA: treatment with 62.5g/l of CDHA combined with 100 micromoles/l of MeJA.

### EXAMPLE 3: Elicitation of Lycopersicon esculentum cells, Micro-Tom variety, with cyclodextrins (CDMA and CDHA) and methyl jasmonate (MeJA) using a low cell density (130g pf/l, 7g ps/λ).

The suspensions of *Lycopersicon esculentum* were treated:
- without CDMA or MeJA (controls)
- with MeJA (100 micromoles/l)
- with CDMA (62.5 g/l)
- with CDMA (62.5 g/l) and MeJA (100 micromoles/l)
- with CDHA (62.5 g/l)
- with CDHA (62.5 g/l) and MeJA (100 micromoles/l)

The experiments were carried out in triplicate in 250 ml volumetric flasks containing 100 ml of culture medium using a low cell density (130 g/pf/l). After 96 hours, the cells and medium were collected separately from each volumetric flask, the cells were weighed and the total quantity of phytosterols in the extracellular medium was determined. The result of the experiment is shown in Table C.

The cultures elicited only with CDs or in combination with MeJA cause the greatest increases in phytosterol production with respect to the controls or even those treated only with MeJA.

In relation to fucosterol and taraxasterol production, it was observed that those cultures treated with CDMA alone or combined with MeJA are those that produce greatest extracellular accumulation.

**TABLE C: Phytosterol production expressed in mg/l of β-sitosterol of Lycopersicon esculentum after 96 hours of incubation. Cell density: (7g ps/l).**

| **Compound** | **Control** | **MeJA** | **CDMA** | **CDMA+MeJA** | **CDHA** | **CDHA+MeJA** |
|---|---|---|---|---|---|---|
| **stigmasterol** | 0 | 3 | 14 | 13 | 13 | 12 |
| **β-sitosterol** | 0 | 3 | 9 | 9 | 7 | 8 |
| **fucosterol** | 4 | 0 | 19 | 34 | 19 | 10 |
| **taraxasterol** | 4 | 8 | 263 | 234 | 6 | 64 |

Control: culture medium without elicitors.
MeJA: treatment with 100 micromoles/l of MeJA.
CDMA: treatment with 62.5g/l of CDMA.
CDMA+MeJA: treatment with 62.5g/l of CDMA combined with 100 micromoles/l of MeJA.
CDHA: treatment with 62.5g/l of CDHA.
CDHA+MeJA: treatment with 62.5g/l of CDHA combined with 100 micromoles/l of MeJA.

### EXAMPLE 4: Elicitation of Rosa sp cells with cyclodextrins (CDMA and CDHA) and methyl jasmonate (MeJA) using a high cell density (420g pf/l, 12.6g ps/l).

The suspensions of *Rosa sp* were treated:
- without CDMA or MeJA (controls)
- with MeJA (100 micromoles/l)
- with CDMA (62.5 g/l)
- with CDMA (62.5 g/l) and MeJA (100 micromoles/l)
- with CDHA (62.5 g/l)
- with CDHA (62.5 g/l) and MeJA (100 micromoles/l)

The experiments were carried out in triplicate in 250 ml volumetric flasks containing 100 ml of culture medium. After 96 hours, the cells and medium were collected separately from each volumetric flask, the cells were weighed and the total quantity of phytosterols in the extracellular medium was determined. The result of the experiment is shown in Table D.

After analysing the results it was observed that the induction of phytosterol biosynthesis in *Rosa sp* cell cultures elicited only with CDs was greater than in that elicited only with MeJA or in combination with CDs, the treatment with CDMA being that which produced the greatest increase.

This fact suggests that the real inducer of β-sitosterol and fucosterol biosynthesis in the *Rosa sp* cultures are the CDs.

**TABLE D: Phytosterol production expressed in mg/l of β-sitosterol of Rosa sp after 96 hours of treatment. Cell density: 12.6g ps/l**

| **Compound** | **Control** | **MeJA** | **CDMA** | **CDMA+MeJA** | **CDHA** | **CDHA**+**MeJA** |
|---|---|---|---|---|---|---|
| **β-sitosterol** | 0 | 4.6 | 167 | 62.5 | 143 | 98.3 |
| **fucosterol** | 0 | 5.1 | 123 | 58 | 150 | 72.8 |

Control: culture medium without elicitors
MeJA: treatment with 100 micromoles/l of MeJA
CDMA: treatment with 62.5g/l of CDMA
CDMA+MeJA: treatment with 62.5g/l of CDMA combined with 100 micromoles/l of MeJA
CDHA: treatment with 62.5g/l of CDHA
CDHA+MeJA: treatment with 62.5g/l of CDHA combined with 100 micromoles/l of MeJA

### EXAMPLE 5: Elicitation of Rosa sp cells with cyclodextrins (CDMA and CDHA) and methyl jasmonate (MeJA) using a low cell density (196g pf/l, 8.4g ps/l).

The suspensions of *Rosa sp* were treated:
- without CDMA or MeJA (controls)
- with MeJA (100 micromoles/l)
- with CDMA (62.5 g/l)
- with CDMA (62.5 g/l) and MeJA (100 micromoles/l)
- with CDHA (62.5 g/l)
- with CDHA (62.5 g/l) and MeJA (100 micromoles/l)

The experiments were carried out in triplicate in 250 ml volumetric flasks containing 100 ml of culture medium. After 96 hours, the cells and medium were collected separately from each volumetric flask, the cells were weighed and the total quantity of phytosterols in the extracellular medium was determined. The result of the experiment is shown in Table E.

The treatment that caused the greatest increase in phytosterol production is CDMA as with the cell cultures of *Rosa sp* elicited using a high cell density (Table D). However in these assays the production of β-sitosterol and fucosterol was in the order of 2 and 3 times less than in the assays performed using a high cell density.

**TABLE E: Phytosterol production expressed in mg/l of β-sitosterol of Rosa sp after 96 hours of treatment. Cell density: 8.4g ps/l**

| **Compound** | **Control** | **MeJA** | **CDMA** | **CDMA+MeJA** | **CDHA** | **CDHA**+**MeJA** |
|---|---|---|---|---|---|---|
| **β-sitosterol** | 0 | 5.5 | 52.5 | 27 | 39.37 | 38.25 |
| **fucosterol** | 0 | 9.8 | 62.5 | 37.12 | 42.62 | 31.62 |

Control: culture medium without elicitors
MeJA: treatment with 100 micromoles/l of MeJA
CDMA: treatment with 62.5g/l of CDMA
CDMA+MeJA: treatment with 62.5g/l of CDMA combined with 100 micromoles/l of MeJA
CDHA: treatment with 62.5g/l of CDHA
CDHA+MeJA: treatment with 62.5g/l of CDHA combined with 100 micromoles/l of MeJA

### EXAMPLE 6: Elicitation of Capsicum annuum cells with cyclodextrins (CDMA) and methyl jasmonate (MeJA) using a high cell density (367g pf/l, 40g ps/l).

The suspensions of *Capsicum annuum* were treated with:
- without CDMA or MeJA (controls)
- with MeJA (100 micromoles/l)
- with CDMA (62.5 g/l)
- with CDMA (62.5 g/l) and MeJA (100 micromoles/l)

The experiments were carried out in triplicate in 250 ml volumetric flasks containing 100 ml of culture medium using a high cell density (367 g/pf/l). After 96 hours, the cells and medium were collected separately from each volumetric flask, the cells were weighed and the total quantity of phytosterols in the extracellular medium was determined. The result of the experiment is shown in Table F.

The induction of phytosterol biosynthesis in cultures elicited only with CDs was less than in those elicited only with MeJA. However, the abundance of phytosterols is greater using the combination of CDs and MeJA than individually for which reason an additive effect is observed.

**TABLE F: Phytosterol production expressed in mg/l of β-sitosterol of Capsicum annuum after 96 hours of incubation. Cell density: 40g ps/l.**

| **Compound** | **Control** | **MeJA** | **CDMA** | **CDMA+ MeJA** |
|---|---|---|---|---|
| **campesterol** | 0 | 16.2 | 9 | 31 |
| **β-sitosterol** | 0 | 81.5 | 67 | 163.8 |

Control: culture medium without elicitors.
MeJA: treatment with 100 micromoles/l of MeJA.
CDMA: treatment with 62.5g/l of CDMA.
CDMA+MeJA: treatment with 62.5g/l of CDMA combined with 100 micromoles/l of MeJA.

### EXAMPLE 7: Elicitation of Capsicum chinense cells with cyclodextrins (CDMA) and methyl jasmonate (MeJA) using a high cell density (367g pf/l, 40g ps/l).

The suspensions of *Capsicum chinense* were treated:
- without CDMA or MeJA (controls)
- with MeJA (100 micromoles/l)
- with CDMA (62.5 g/l)
- with CDMA (62.5 g/l) and MeJA (100 micromoles/l)

The experiments were carried out in triplicate in 250 ml volumetric flasks containing 100 ml of culture medium using a high cell density (367 g/pf/l). After 96 hours, the cells and medium were collected separately from each volumetric flask, the cells were weighed and the total quantity of phytosterols in the extracellular medium was determined. The result of the experiment is shown in Table G.

As is observed in table G, β-sitosterol biosynthesis is increased on performing the assays with CDs alone or in combination with MeJA whilst the elicitation in the presence of MeJA individually produces lower levels. No production of campesterol, fucosterol and stigmasterol were detected in the presence of MeJA but they were with CDs and the combination of both.

**TABLE G: Phytosterol production expressed in mg/l of β-sitosterol of Capsicum chinense after 96 hours of incubation. Cell density: 40g ps/l.**

| **COMPOUND** | **CONTROL** | **MeJA** | **CDMA** | **CDMA+MeJA** |
|---|---|---|---|---|
| **stigmasterol** | 0 | 0 | 22 | 20 |
| **β-sitosterol** | 0 | 8.4 | 137 | 141.1 |
| **campesterol** | 0 | 0 | 46 | 33.25 |
| **fucosterol** | 0 | 0 | 51.6 | 25.6 |

Control: culture medium without elicitors.
MeJA: treatment with 100 micromoles/l of MeJA.
CDMA: treatment with 62.5g/l of CDMA.
CDMA+MeJA: treatment with 62.5g/l of CDMA combined with 100 micromoles/l of MeJA.

### EXAMPLE 8: Elicitation of cells of Daucus carota L. sativus (Hoffm.) Arcang PC-1106 with cyclodextrins (CDMA and CDHA) and methyl jasmonate (MeJA) using a low cell density (165g pf/l, 5g ps/l).

The suspensions of *Daucus carota* L. sativus (Hoffm.) Arcang PC-1106 were treated with:
- without CDMA or MeJA (controls)
- with MeJA (100 micromoles/l)
- with CDMA (62.5 g/l)
- with CDMA (62.5 g/l) and MeJA (100 micromoles/l)
- with CDHA (62.5 g/l)
- with CDHA (62.5 g/l) and MeJA (100 micromoles/l)

The experiments were carried out in triplicate in 250 ml volumetric flasks containing 100 ml of culture medium using a low cell density (11.2 g pf/l). After 96 hours, the cells and medium were collected separately from each volumetric flask, the cells were weighed and the total quantity of phytosterols in the extracellular medium was determined. The result of the experiment is shown in Table H.

After analysing the results it was observed that both stigmasterol and campesterol and β-sitosterol were identified in those cultures treated only with CDs or in combination with MeJA.

The induction of the phytosterol biosynthesis in cultures elicited with CDMA+MeJA was similar to those performed only in the presence of CDMA.

**TABLE H: Phytosterol production expressed in mg/l of β-sitosterol of Daucus carota L. sativus (Hoffm.) Arcang PC-1106 after 96 hours of incubation. Cell**

| **COMPOUND** | **CONTROL** | **MeJA** | **CDMA** | **CDMA+MeJA** | **CDHA** | **CDHA**+**MeJA** |
|---|---|---|---|---|---|---|
| **stigmasterol** | 0 | 0 | 3.6 | 4.3 | 3.7 | 4.9 |
| **β-sitosterol** | 0 | 0 | 3.3 | 3.8 | 3.6 | 2.7 |
| **campesterol** | 0 | 0 | 3.2 | 3.6 | 3.5 | 3.4 |

density: 5g ps/l
Control: culture medium without elicitors.
MeJA: treatment with 100 micromoles/l of MeJA.
CDMA: treatment with 62.5g/l of CDMA.
CDMA+MeJA: treatment with 62.5g/l of CDMA combined with 100 micromoles/l of MeJA.
CDHA: treatment with 62.5g/l of CDHA.
CDHA+MeJA: treatment with 62.5g/l of CDHA combined with 100 micromoles/l of MeJA.

### EXAMPLE 9: Elicitation of cells of Daucus carota L. sativus (Hoffm.) Arcang PC-1106 with cyclodextrins (CDMA and CDHA) and methyl jasmonate (MeJA) using a high cell density (330g pf/l, 11.2g ps/l).

The suspensions were treated:
- without CDMA or MeJA (controls)
- with MeJA (100 micromoles/l)
- with CDMA (62.5 g/l)
- with CDMA (62.5 g/l) and MeJA (100 micromoles/l)
- with CDHA (62.5 g/l)
- with CDHA (62.5 g/l) and MeJA (100 micromoles/l)

The experiments were carried out in triplicate in 250 ml volumetric flasks containing 100 ml of culture medium using a low cell density (11.2 g pf/l). After 96 hours, the cells and medium were collected separately from each volumetric flask, the cells were weighed and the total quantity of phytosterols in the extracellular medium was determined. The result of the experiment is shown in Table I.

The same as what occurred with the elicitation experiments of *Daucus carota* L. sativus cells using a low cell density (Table H), stigmasterol, campesterol and β-sitosterol were only identified in those cultures treated with CDs individually or in combination with MeJA, the production levels of phytosterols being similar in all the treatments.

**TABLE I: Phytosterol production expressed in mg/l of β-sitosterol of Daucus carota L. sativus (Hoffm.) Arcang PC-1106 after 96 hours of incubation. Cell**

| **COMPOUND** | **CONTROL** | **MeJA** | **CDMA** | **CDMA+MeJA** | **CDHA** | **CDHA+MeJA** |
|---|---|---|---|---|---|---|
| **stigmasterol** | 0 | 0 | 6.4 | 5.7 | 4.7 | 5.4 |
| **β-sitosterol** | 0 | 0 | 6.1 | 5.0 | 4.7 | 5.0 |
| **campesterol** | 0 | 0 | 5.5 | 4.4 | 4.5 | 4.7 |

density: 11.2g ps/l
Control: culture medium without elicitors.
MeJA: treatment with 100 micromoles/l of MeJA.
CDMA: treatment with 62.5g/l of CDMA.
CDMA+MeJA: treatment with 62.5g/l of CDMA combined with 100 micromoles/l of MeJA.
CDHA: treatment with 62.5g/l of CDHA.
CDHA+MeJA: treatment with 62.5g/l of CDHA combined with 100 micromoles/l of MeJA.

### EXAMPLE 10: Elicitation of Daucus carota cells, Chantenay variety with cyclodextrins (CDMA and CDHA) and methyl jasmonate (MeJA) using a high cell density (270g pf/l, 16.5g ps/l).

The suspensions of *Daucus carota,* Chantenay variety, were treated:
- controls without CDMA or MeJA
- with MeJA (100 micromoles/l)
- with CDMA (62.5 g/l)
- with CDMA (62.5 g/l) and MeJA (100 micromoles/l)
- with CDHA (62.5 g/l)
- with CDHA (62.5 g/l) and MeJA (100 micromoles/l)

The experiments were carried out in triplicate in 250 ml volumetric flasks containing 100 ml of culture medium. After 96 hours, the cells and medium were collected separately from each volumetric flask, the cells were weighed and the total quantity of phytosterols in the extracellular medium was determined. The result of the experiment is shown in Table J.

These results indicate that phytosterol production in this cell line is similar in all the treatments with CDs only or in combination with MeJA.

**TABLE J: Phytosterol production expressed in mg/l of β-sitosterol of Daucus carota, Chantenay variety, after 96 hours of treatment. Cell density: 16.5g ps/l**

| **Compound** | **Control** | **MeJA** | **CDMA** | **CDMA+MeJA** | **CDHA** | **CDHA**+**MeJA** |
|---|---|---|---|---|---|---|
| **stigmasterol** | 0 | 0 | 3.46 | 4.16 | 3.22 | 3.87 |
| **β-sitosterol** | 0 | 0 | 3.41 | 3.55 | 3.22 | 3.27 |

Control: culture medium without elicitors
MeJA: treatment with 100 micromoles/l of MeJA
CDMA: treatment with 62.5g/l of CDMA
CDMA+MeJA: treatment with 62.5g/l of CDMA combined with 100 micromoles/l of MeJA
CDHA: treatment with 62.5g/l of CDHA
CDHA+MeJA: treatment with 62.5g/l of CDHA combined with 100 micromoles/l of MeJA

### EXAMPLE 11: Elicitation of Vitis vinifera cells cv Monastrell with cyclodextrins (CDMA) and methyl jasmonate (MeJA) using a high cell density (300g pf/l, 2.5g ps/l).

The suspensions of *Vitis vinifera* were treated:
- without CDMA or MeJA (controls)
- with MeJA (100 micromoles/l)
- with CDMA (62.5 g/l)
- with CDMA (62.5 g/l) and MeJA (100 micromoles/l)
- with CDHA (62.5 g/l)
- with CDHA (62.5 g/l) and MeJA (100 micromoles/l)

The experiments were carried out in triplicate in 250 ml volumetric flasks containing 100 ml of culture medium using a high cell density (300 g/pf/l). After 96 hours, the cells and medium were collected separately from each volumetric flask, the cells were weighed and the total quantity of phytosterols in the extracellular medium was determined. The result of the experiment is shown in Table K.

Phytosterols biosynthesis occurred on performing the assays with CDs whilst in the cell cultures treated only with MeJA no extracellular accumulation occurred.

**TABLE K: Phytosterol production expressed in mg/l of β-sitosterol of Vitis vinifera cv Monastrell after 96 hours of incubation. Cell density: 2.5g ps/l.**

| **COMPOUND** | **CONTROL** | **MeJA** | **CDMA** | **CDMA+MeJA** |
|---|---|---|---|---|
| **β-sitosterol** | 0 | 0 | 37.4 | 31.6 |
| **fucosterol** | 0 | 0 | 7.5 | 0 |

Control: culture medium without elicitors
MeJA: treatment with 100 micromoles/l
CDMA: treatment with 62.5g/l
CDMA+MeJA: treatment with 62.5g/l of CDMA combined with 100 micromoles/l of MeJA
CDHA: treatment with 62.5g/l of CDHA
CDHA+MeJA: treatment with 62.5g/l of CDHA combined with 100 micromoles/l of MeJA

### EXAMPLE 12: Elicitation of Catharanthus roseus (L.) G.Don cells with cyclodextrins (CDMA and CDHA) and methyl jasmonate (MeJA) using a high cell density (232g pf/l, 9.2g ps/l).

The suspensions of *Catharanthus roseus* (L.) G. Don were treated with:
- without CDMA or MeJA (controls)
- with MeJA (100 micromoles/l)
- with CDMA (62.5 g/l)
- with CDMA (62.5 g/l) and MeJA (100 micromoles/l)
- with CDHA (62.5 g/l)
- with CDHA (62.5 g/l) and MeJA (100 micromoles/l)

The experiments were carried out in triplicate in 250 ml volumetric flasks containing 100 ml of culture medium using a low cell density (11.2 g pf/l). After 96 hours, the cells and medium were collected separately from each volumetric flask, the cells were weighed and the total quantity of phytosterols in the extracellular medium was determined. The result of the experiment is shown in Table L.

The cultures elicited only with CDs or in combination with MeJA caused phytosterol production. The assays performed in the presence of CDs caused an increase in extracellular accumulation of phytosterols. In all cases, a low phytosterol production was observed, for which purpose the effect or the inducing agents are the CDs.

**TABLE L: Phytosterol production expressed in mg/l of β-sitosterol of Catharanthus roseus (L.) G.Don after 96 hours of incubation. Cell density: 9.2g ps/l**

| **COMPOUND** | **CONTROL** | **MeJA** | **CDHA** | **CDHA+MeJA** |
|---|---|---|---|---|
| **campesterol** | 0 | 0 | 3.3 | 3.4 |
| **β-sitosterol** | 0 | 0 | 2.8 | 0 |

Control: culture medium without elicitors.
MeJA: treatment with 100 micromoles/l of MeJA.
CDMA: treatment with 62.5g/l of CDMA.
CDMA+MeJA: treatment with 62.5g/l of CDMA combined with 100 micromoles/l of MeJA.
CDHA: treatment with 62.5g/l of CDHA.
CDHA+MeJA: treatment with 62.5g/l of CDHA combined with 100 micromoles/l of MeJA.

### EXAMPLE 13: Elicitation of Catharanthus roseus (L.) G.Don cells with cyclodextrins (CDMA and CDHA) and methyl jasmonate (MeJA) using a low cell density (110g pf/l, 6g ps/l).

The suspensions of *Catharanthus roseus* (L.) G.Don were treated with:
- without CDMA or MeJA (controls)
- with MeJA (100 micromoles/l)
- with CDMA (62.5 g/l)
- with CDMA (62.5 g/l) and MeJA (100 micromoles/l)
- with CDHA (62.5 g/l)
- with CDHA (62.5 g/l) and MeJA (100 micromoles/l)

The experiments were carried out in triplicate in 250 ml volumetric flasks containing 100 ml of culture medium using a low cell density (110 g pf/l). After 96 hours, the cells and medium were collected separately from each volumetric flask, the cells were weighed and the total quantity of phytosterols in the extracellular medium was determined. The result of the experiment is shown in Table M.

Only the cell cultures elicited with CDs increased campesterol and β-sitosterol production with respect to the other treatments. Therefore, in *Catharanthus roseus* (L.) G.Don the CDs produce an increase in phytosterol biosynthesis.

**TABLE M: Phytosterol production expressed in mg/l of β-sitosterol I of Catharanthus roseus (L.) G.Don after 96 hours of incubation. Cell density: 6g ps/l**

| **COMPOUND** | **CONTROL** | **MeJA** | **CDMA** | **CDMA+MeJA** | **CDHA** | **CDHA+MeJA** |
|---|---|---|---|---|---|---|
| **β-sitosterol** | 0 | 0 | 4.7 | 0 | 3.29 | 0 |
| **campesterol** | 0 | 0 | 3.86 | 0 | 2.78 | 0 |

Control: culture medium without elicitors.
MeJA: treatment with 100 micromoles/l of MeJA.
CDMA: treatment with 62.5g/l of CDMA.
CDMA+MeJA: treatment with 62.5g/l of CDMA combined with 100 micromoles/l of MeJA.
CDHA: treatment with 62.5g/l of CDHA.
CDHA+MeJA: treatment with 62.5g/l of CDHA combined with 100 micromoles/l of MeJA.

### EXAMPLE 14: Elicitation of Lactuca sativa cells obtained from leaves with cyclodextrins (CDMA and CDHA) and methyl jasmonate (MeJA) using a high cell density (250g pf/l, 3g ps/l).

The suspensions of *Lactuca sativa* were treated:
- without CDMA or MeJA (controls)
- with MeJA (100 micromoles/l)
- with CDMA (62.5 g/l)
- with CDMA (62.5 g/l) and MeJA (100 micromoles/l)
- with CDHA (62.5 g/l)
- with CDHA (62.5 g/l) and MeJA (100 micromoles/l)

The experiments were carried out in triplicate in 250 ml volumetric flasks containing 100 ml of culture medium. After 96 hours, the cells and medium were collected separately from each volumetric flask, the cells were weighed and the total quantity of phytosterols in the extracellular medium was determined. The result of the experiment is shown in Table N.

The cultures elicited only with CDs or in combination with MeJA, induce greatest extracellular accumulation of phytosterols, the treatment with CDMA being that which produced the greatest increase in production with respect to the control and the cultures treated only with MeJA.

On the other hand, it was observed that there were no significant differences in phytosterol production in the assays performed with CDMA+MeJA, CDHA and CDHA+MeJA.

**TABLE N: Phytosterol production expressed in mg/l of β-sitosterol of Lactuca sativa after 96 hours of treatment. Cell density: 2.8g ps/l**

| **Compound** | **Control** | **MeJA** | **CDMA** | **CDMA+MeJA** | **CDHA** | **CDHA**+**MeJA** |
|---|---|---|---|---|---|---|
| **campesterol** | 0 | 0 | 5.36 | 3.18 | 3.17 | 3.11 |
| **stigmasterol** | 0 | 0 | 6.05 | 3.63 | 4.31 | 4.4 |
| **β-sitosterol** | 0 | 0 | 5.63 | 3.66 | 3.43 | 3.33 |

Control: culture medium without elicitors
MeJA: treatment with 100 micromoles/l of MeJA
CDMA: treatment with 62.5g/l of CDMA
CDMA+MeJA: treatment with 62.5g/l of CDMA combined with 100 micromoles/l of MeJA
CDHA: treatment with 62.5g/l of CDHA
CDHA+MeJA: treatment with 62.5g/l of CDHA combined with 100 micromoles/l of MeJA

### EXAMPLE 15: Elicitation of Lactuca sativa cells obtained from the root with cyclodextrins (CDMA and CDHA) and methyl jasmonate (MeJA) using a high cell density (250g pf/l, 3g ps/l).

The suspensions of *Lactuca sativa* were treated:
- without CDMA or MeJA (controls)
- with MeJA (100 micromoles/l)
- with CDMA (62.5 g/l)
- with CDMA (62.5 g/l) and MeJA (100 micromoles/l)
- with CDHA (62.5 g/l)
- with CDHA (62.5 g/l) and MeJA (100 micromoles/l)

The experiments were carried out in triplicate in 250 ml volumetric flasks containing 100 ml of culture medium. After 96 hours, the cells and medium were collected separately from each volumetric flask, the cells were weighed and the total quantity of phytosterols in the extracellular medium was determined. The result of the experiment is shown in Table N.

The same as what occurred with the elicitation experiments of *Lactuca sativa* cells obtained from leave (Table N), stigmasterol, campesterol and β-sitosterol were only identified in those cultures treated with CDs individually or in combination with MeJA, the production level of phytosterols being similar in all treatments.

**TABLE N: Phytosterol production expressed in mg/l of β-sitosterol of Lactuca sativa after 96 hours of treatment. Cell density: 3g ps/l**

| **Compound** | **Control** | **MeJA** | **CDMA** | **CDMA+MeJA** | **CDHA** | **CDHA**+**MeJA** |
|---|---|---|---|---|---|---|
| **campesterol** | 0 | 0 | 5.53 | 4.41 | 4.51 | 2.17 |
| **stigmasterol** | 0 | 0 | 6.45 | 5.70 | 4.72 | 5.13 |
| **β-sitosterol** | 0 | 0 | 6.12 | 4.96 | 4.71 | 5.02 |

Control: culture medium without elicitors
MeJA: treatment with 100 micromoles/l of MeJA
CDMA: treatment with 62.5g/l of CDMA
CDMA+MeJA: treatment with 62.5g/l of CDMA combined with 100 micromoles/l of MeJA
CDHA: treatment with 62.5g/l of CDHA
CDHA+MeJA: treatment with 62.5g/l of CDHA combined with 100 micromoles/l of MeJA

## Claims

1. Process to increase the production and/or extraction of phytosterols comprising:
a. adding cyclodextrins to a culture medium,
b. contacting plant cells with the potential to produce phytosterol with the culture medium of (a),
c. incubating the cells of step b) in the culture medium of step a),
d. separating the phytosterols obtained after step c) from the culture medium.

2. Process according to the preceding claim, where the plant cells with the potential to produce phytosterol of step (a) come from plants of the genera selected from the list comprising: *Rosa, Daucus, Capsicum, Lactuca, Catharanthus, Lycopersicon, Taxus* and *Vitis.*

3. Process according to any of the preceding claims, where the plant cells with the potential to produce phytosterol come from vitroplants.

4. Process according to any of claims 1-3, where the cyclodextrins are selected from the group comprising randomly methylated cyclodextrin or hydroxypropylated cyclodextrin.

5. Process according to claim 4, where the randomly methylated cyclodextrin has a degree of substitution by methyls of between 1 and 3.

6. Process according to claim 5, where the randomly methylated cyclodextrin is dimethylated.

7. Process according to claim 4, where the randomly hydroxypropylated cyclodextrin has a degree of substitution by hydroxypropyls of between 0.6 and 0.9.

8. Process according to any of claims 1 to 7, where the cyclodextrin is a β-cyclodextrin.

9. Process according to any of claims 1 to 8, where the concentration of cyclodextrins in the culture medium of step (a) is between 6.5 and 130 g/l.

10. Process according to any of claims 1 to 9, where the concentration of cyclodextrins in the culture medium of step (a) is between 10 and 100 g/l.

11. Process according to any of claims 1 to 10 where the concentration of cyclodextrins in the culture medium of step (a) is between 50 and 75 g/l.

12. Process according to any of claims 1 to 11, where, in addition to adding cyclodextrins to the culture medium of step (a), methyl jasmonate is added.

13. Process according to claim 12, where the concentration of methyl jasmonate in the culture medium of step (a) is between 5 and 500 micromoles/ litre.

14. Process according to any of claims 12-13, where the concentration of methyl jasmonate in the culture medium of step (a) is between 25 and 150 micromoles/ litre.

15. Process according to any of claims 12-14, where the concentration of methyl jasmonate in the culture medium of step (a) is between 75 and 125 micromoles/ litre.

16. Use of a composition comprising cyclodextrins to increase the production and/or extraction of phytosterols in cells with the potential to produce phytosterol.

17. Use of a culture medium comprising cyclodextrins to increase the production and/or extraction of phytosterols in cells with the potential to produce phytosterol.

18. Use of a composition according to claim 16 or of a culture medium according to claim 17, where the composition or the culture medium further comprises methyl jasmonate.

## Patentansprüche

1. Verfahren zur Erhöhung der Herstellung und/oder Extraktion von Phytosterolen, das Folgendes umfasst:
a. Versetzen eines Kulturmediums mit Cyclodextrinen,
b. Kontaktieren von Pflanzenzellen mit Potenzial zur Herstellung von Phytosterol mit dem Kulturmedium aus Schritt a),
c. Inkubieren der Zellen aus Schritt b) im Kulturmedium aus Schritt a),
d. Abtrennen der nach Schritt c) erhaltenen Phytosterole aus dem Kulturmedium.

2. Verfahren nach dem vorhergehenden Anspruch, wobei die Pflanzenzellen mit Potenzial zur Herstellung von Phytosterol aus Schritt a) von Pflanzen der Gattungen stammen, die aus der Liste, umfassend *Rosa, Daucus, Capsicum, Lactuca, Catharanthus, Lycopersicon, Taxus* und *Vitis,* ausgewählt werden.

3. Verfahren nach einem der vorhergehenden Ansprüche, wobei die Pflanzenzellen mit Potenzial zur Herstellung von Phytosterol von Vitro-Pflanzen stammen.

4. Verfahren nach einem der Ansprüche 1 bis 3, wobei die Cyclodextrine ausgewählt werden aus der Gruppe, die willkürlich methyliertes Cyclodextrin oder hydroxypropyliertes Cyclodextrin umfasst.

5. Verfahren nach Anspruch 4, wobei das willkürlich methylierte Cyclodextrin einen Substitutionsgrad durch Methyl von 1 bis 3 aufweist.

6. Verfahren nach Anspruch 5, wobei das willkürlich methylierte Cyclodextrin dimethyliert ist.

7. Verfahren nach Anspruch 4, wobei das willkürlich hydroxypropylierte Cyclodextrin einen Substitutionsgrad durch Hydroxypropyl von 0,6 bis 0,9 aufweist.

8. Verfahren nach einem der Ansprüche 1 bis 7, wobei das Cyclodextrin ein β-Cyclodextrin ist.

9. Verfahren nach einem der Ansprüche 1 bis 8, wobei die Konzentration der Cyclodextrine im Kulturmedium aus Schritt (a) zwischen 6,5 und 130 g/l liegt.

10. Verfahren nach einem der Ansprüche 1 bis 9, wobei die Konzentration der Cyclodextrine im Kulturmedium aus Schritt (a) zwischen 10 und 100 g/l liegt.

11. Verfahren nach einem der Ansprüche 1 bis 10, wobei die Konzentration der Cyclodextrine im Kulturmedium aus Schritt (a) zwischen 50 und 75 g/l liegt.

12. Verfahren nach einem der Ansprüche 1 bis 11, wobei zusätzlich zur Zugabe von Cyclodextrinen zum Kulturmedium aus Schritt (a) Methyljasmonat hinzugefügt wird.

13. Verfahren nach Anspruch 12, wobei die Konzentration von Methyljasmonat im Kulturmedium aus Schritt (a) zwischen 5 und 500 Mikromol/Liter liegt.

14. Verfahren nach einem der Ansprüche 12 bis 13, wobei die Konzentration von Methyljasmonat im Kulturmedium aus Schritt (a) zwischen 25 und 150 Mikromol/Liter liegt.

15. Verfahren nach einem der Ansprüche 12 bis 14, wobei die Konzentration von Methyljasmonat im Kulturmedium aus Schritt (a) zwischen 75 und 125 Mikromol/Liter liegt.

16. Verwendung einer Cyclodextrine umfassenden Zusammensetzung zur Erhöhung der Herstellung und/oder Extraktion von Phytosterolen in Zellen mit Potenzial zur Herstellung von Phytosterol.

17. Verwendung eines Cyclodextrine umfassenden Kulturmediums zur Erhöhung der Herstellung und/oder Extraktion von Phytosterolen in Zellen mit Potenzial zur Herstellung von Phytosterol.

18. Verwendung einer Zusammensetzung nach Anspruch 16 oder eines Kulturmediums nach Anspruch 17, wobei die Zusammensetzung oder das Kulturmedium weiterhin Methyljasmonat umfasst.

## Revendications

1. Un procédé pour augmenter la production et/ou l'extraction de phytostérols qui comprend :
a) addition de cyclodextrines au milieu de culture,
b) mettre en contact des cellules végétales ayant la capacité de produire du phytostérol avec le milieu de culture d' (a),
c) incubation des cellules de l'étape b) dans le milieu de culture de l'étape a),
d) séparation des phytostérols obtenus après l'étape c) du milieu de culture.

2. Un procédé selon la revendication précédente, dans lequel les cellules végétales ayant la capacité de produire des phytostérols de l'étape (a) proviennent des plantes des genres sélectionnés parmi la liste comprenant : *Rosa, Daucus, Capsicum. Lactuca, Catharanthus, Lycopersicon, Taxus* et *Vitis.*

3. Un procédé selon l'une des revendications précédentes, dans lequel les cellules végétales ayant la capacité de produire des phytostérols proviennent des vitroplants.

4. Un procédé selon l'une des revendications 1-3, dans lequel les cyclodextrines sont sélectionnées parmi le groupe comprenant des cyclodextrines méthylées ou des cyclodextrines hydroxypropylées aléatoirement.

5. Un procédé selon la revendication 4, dans lequel la cyclodextrine méthylée aléatoirement présente un degré de substitution de méthyles entre 1 et 3.

6. Un procédé selon la revendication 5, dans lequel la cyclodextrine méthylée est diméthylée.

7. Un procédé selon la revendication 4, dans lequel la cyclodextrine hydroxypropylée aléatoirement présente un degré de substitution des hydroxypropyles d'entre 0,6 et 0,9.

8. Un procédé selon l'une des revendications 1 à 7, dans lequel la cyclodextrine est une β -cyclodextrine.

9. Un procédé selon l'une des revendications 1 à 8, dans lequel la concentration des cyclodextrines dans le milieu de culture de l'étape (a) se trouve entre 6,5 et 130 g/l.

10. Un procédé selon l'une des revendications 1 à 9, dans lequel la concentration des cyclodextrines dans le milieu de culture de l'étape (a) se trouve entre 10 et 100 g/l.

11. Un procédé selon l'une des revendications 1 à 10, dans lequel la concentration des cyclodextrines dans le milieu de culture de l'étape (a) se trouve entre 50 et 75 g/l.

12. Un procédé selon l'une des revendications 1 à 11, dans lequel, en plus de l'addition des cyclodextrines au milieu de culture de l'étape (a), on ajoute du méthyle jasmonate.

13. Un procédé selon la revendication 12, dans lequel la concentration de méthyle jasmonate dans le milieu de culture de l'étape (a) se trouve entre 5 et 500 micromoles/litre.

14. Un procédé selon l'une des revendications 12-13, dans lequel la concentration du méthyle jasmonate dans le milieu de culture de l'étape (a) se trouve entre 25 et 150 micromoles/litre.

15. Un procédé selon l'une des revendications 12-14, dans lequel la concentration du méthyle jasmonate dans le milieu de culture de l'étape (a) se trouve entre 75 et 125 micromoles/litre.

16. Utilisation d'une composition comprenant des cyclodextrines pour augmenter la production et/ou extraction des phytostérols des cellules ayant la capacité de produire du phytostérol.

17. Utilisation d'un milieu de culture comprenant des cyclodextrines pour augmenter la production et/ou extraction de phytostérols des cellules ayant la capacité de produire du phytostérol.

18. Utilisation d'une composition selon la revendication 16 ou d'un milieu de culture selon la revendication 17, dans laquelle la composition ou le milieu de culture comprend en plus du méthyle jasmonate.
